# EUROPEAN PATENT APPLICATION

(11) **EP 1 932 925 A1**
(43) Date of publication of application: **18.06.2008**
(21) Application number: 07023440.6
(22) Date of filing: 04.12.2007
(51) Int. Cl.: C12Q 1/68

(54) **Method for detecting mutation of nucleic acid using single-stranded DNA-binding protein**

(30) Priority: 04.12.2006 JP 2006327475
(71) Applicant: FUJIFILM Corporation, Minato-ku Tokyo (JP)
(72) Inventor: Iwaki, Yoshihide, Ashigarakami-gun Kanagawa (JP); Mori, Toshihiro, Ashigarakami-gun Kanagawa (JP); Ishii, Yasuyuki, Ashigarakami-gun Kanagawa (JP); Yoshida, Junya, Ashigarakami-gun Kanagawa (JP)
(74) Representative: HOFFMANN EITLE

(57) **Abstract**

A method for judging the presence or absence of a mutation in a nucleic acid sequence, the method includes utilizing a single-stranded DNA-binding protein; the aforementioned method for judging the presence or absence of a mutation in a nucleic acid sequence, wherein the aforementioned presence or absence of a mutation in a nucleic acid sequence is judged by a product formed by a nucleic acid amplification reaction utilizing the single-stranded DNA-binding protein; and a kit for judging the presence or absence of the aforementioned mutation in a nucleic acid sequence.

## Description

### Background of the Invention

### 1. Field of the Invention

This invention relates to a method for detecting a mismatch in double-stranded nucleic acid characterized by the use of single-stranded DNA-binding protein. According to the method of the invention, single nucleotide polymorphism (SNPs; also called single nucleotide substitution or SNP) in DNA nucleotide sequences can be detected.

### 2. Description of the Related Art

Gene expression profile analysis and analysis of SNPs in genes are drawing attention followed by the genomic sequence analysis. Gene functions and relationships between genes and diseases or medicament sensitivities have been examined by the analysis of genes expressing under various conditions, gene mutations in various individuals and the like. In addition, diagnosis of diseases and the like have recently been carried out using knowledge on these genes.

Detection of mutation in nucleic acid sequences is very important in the field of medical genetics. Detection of genetic mutations is important for the determination of molecular biological basis of hereditary diseases, provision of carriers and prenatal diagnoses for hereditary counseling, acceleration of individualization in medicines, identification of polymorphism in genetic studies and the like.

The method for typing conventionally known SNPs is divided into a method which uses polymerase reaction and a method which uses hybridization when classified based on the principle.

There are three methods regarding the method which uses hybridization, namely the simple hybridization which uses a DNA chip (Sequence By Hybridization) (Drmanac R. et al., Genomics, 4, 114 - 128 (1989)), the Dye-labeled oligonucleotide ligation method (Chen X. et al., Genome Res., 8, 549 - 556 (1998)) and the Invader method (Lyamichev et al., Science, 260, 778 - 783 (1993)). In each case, the principle is to prepare oligonucleotides corresponding to respective alleles and thereby detect hybridization with which allele. Since these methods have a problem in that a period of time is required because of the necessity to carry out a hybridization operation, or the device is expensive due to the employment of fluorescence in the detection system, so that the inspection cannot be carried out conveniently.

The method which uses polymerase reaction is divided into a method in which a primer is set to a vicinity of an SNP and incorporation of a base at the SNP site is detected, such as the SNaPShot method and Pyrosequence method (Alderborn, A. et al., Genome Res., 28, 1249 - 1258 (2000)) and a method in which a primer is designed in such a manner that an SNP site which corresponds to each allele is contained in the vicinity of the 3' end and the judgment is carried out based on whether or not the polymerase reaction occurs (ARMS method (Amplification refractory mutation system), Newton CR. et al., Nucl. Acids Res., 17, 2503 - 2516 (1989)), (PASA method (PCR-amplification of specific alleles), Sarker G. et al., Anal. Biochem., 186, 64 - 68 (1990)).

The SNaPShot method is a method in which a primer is prepared until just before the SNP site to carry out the elongation reaction by a dideoxynucleotide alone and which base is incorporated is analyzed. Being an elongation reaction of only 1 base, it is necessary to use a sequencer so that there is a problem of requiring an expensive device.

The ARMS method and PASA method make use of the strong dependency of the elongation reaction as the starting point of the primer on the matching of the primer 3'-end with the template (Kwok S. et al., Nucleic Acids Res., 18, 999 - 1005 (1990), Huang M. M. et al., Nucleic Acids Res., 20, 4567 - 4573 (1992)). That is, these are methods in which primers complementary to respective alleles are prepared in advance and the genetic type is judged by whether or not the amplification reaction occurred, making used of the fact that the elongation reaction occurs only when coincided with the genetic type of the sample.

However, in reality, there is a difference of only one base between respective allele-specific primers so that a nonspecific amplification occurs sometimes by a mismatch primer (Huang M.M. et al., Nucleic Acids Res., 20, 4567 - 4573 (1992)). In addition, since whether or not the amplification occurs is influenced also by delicate conditions such as of the device to be used, the peripheral environment and the like, it is difficult to suppress the nonspecific amplification.

Discrimination of primers by their difference in one base in a reaction system which requires a temperature cycle, particularly typified by the PCR method, causes further more difficulty in suppressing the nonspecific amplification when the great influence of temperature upon the hydrogen bond of base pairs is taken into consideration.

Also, a method for detecting a mismatch formed in a hybridization product has recently been proposed by the use of a labeled mismatch recognizingprotein, MutS protein, for the purpose of improving specificity of the method which uses hybridization (JP-A-2003-52396). However, the problem in that a period of time is required because of the requirement of hybridization operation or that the device is expensive due to the employment of fluorescence in the detection system has not been solved. In addition, it is known that binding strength of the MutS protein varies depending on the kind of mismatch. Particularly, its binding for a pyrimidine-pyrimidine mismatch is weak (M. Gotoh et al., Genet. Anal., 14, 47 - 50 (1997)).

Accordingly, when a mismatch recognizing protein is applied to the SNPs typing, it poses a problem in that a danger of overlooking a mutation is high.

### Summary of the Invention

The invention aims at providing a method for detecting a mismatch in a double stranded nucleic acid more efficiently and more accurately.

With the aim of solving the aforementioned problems, the present inventors have conducted intensive examinations and found as a result that amplification of a nucleic acid in which a mismatch is present can be selectively suppressed by allowing it to contact with a single-stranded DNA-binding protein at the time of the nucleic acid amplification. This is a surprising fact which has not been known as an effect of the single-stranded DNA-binding protein. The invention has been accomplished based on such knowledge.

That is, the invention consists of the following constructions.
<1> A method for judging presence or absence of a mutation in a nucleic acid sequence, the method comprising:
   utilizing a single-stranded DNA-binding protein.
<2> The method as described in <1> above, comprising:
   (a) obtaining an amplification reaction solution by allowing a target nucleic acid, a primer and a reagent capable of amplifying the target nucleic acid and the single-stranded DNA-binding protein to contact with one another;
   (b) amplifying the target nucleic acid by elongating the primer; and
   (c) judging presence or absence of a mismatch between a control nucleic acid and the target nucleic acid by detecting whether or not the target nucleic acid is amplified.
<3> The method as described in <1> or <2> above,
   wherein the presence or absence of a mutation in a nucleic acid sequence is judged by a product formed by a nucleic acid amplification reaction utilizing the single-stranded DNA-binding protein.
<4> The method as described in any of <1> to <3> above,
   wherein the presence or absence of a mutation in a nucleic acid sequence is judged by presence or absence of an amplification reaction based on an action of the single-stranded DNA-binding protein to suppress a non-specific reaction of the nucleic acid amplification reaction.
<5> The method as described in any of <2> to <4> above,
   wherein the nucleic acid amplification reaction is isothermally carried out.
<6> The method as described in any of <2> to <5> above,
   wherein the nucleic acid amplification reaction is carried out utilizing a strand displacement type polymerase.
<7> The method as described in any of <1> to <6> above,
   wherein the single-stranded DNA-binding protein is an SSB derived from *Escherichia coli, Drosophila* or *Xenopus,* a T4 phage gene 32, 41, 44, 45 or 61 protein or a mixture of at least two species thereof.
<8> The method as described in any of <1> to <7> above,
   wherein a series of actions of extracting, amplifying and detecting a nucleic acid from a substance to be tested is continuously carried out in a sealed space.
<9> The method as described in <8> above,
   wherein the substance to be tested is selected from the group consisting of blood, body fluids, tissues, cells, bacteria and viruses.
<10> A kit for judging presence or absence of a mutation in a nucleic acid sequence by the method as described in any of <1> to <9> above.

### Brief Description of the Drawings

Fig. 1 is a graph describing physical relationship of respective primers in Example 1. In this connection, the β-actin gene is double-stranded, and the upper row double strand and the lower row double strand are respectively connected to the upper row right end and lower row left end. The left end of the upper side of each of the upper row and lower row is 3'-end and the left end of the lower side of each of the upper row and lower row is 5' -end. In addition, each blank between bases of the DNA sequence is for the sake of convenience and they are continued in reality;
Fig. 2 is a graph showing a result of Example 1;
Fig. 3 is a graph describing physical relationship of respective primers in Example 2. In this connection, arrangement of the double-stranded gene is the same as that of Fig. 1;
Fig. 4 is a graph showing a result of Example 2; and
Fig. 5 is a graph showing a result of Example 3.

### Detailed Description of the Invention

This invention relates to a method for detecting a single strand of a mismatch moiety possessed by a double-stranded nucleic acid making use of a single-stranded DNA-binding protein. The method of the invention is a method which uses the ability of the single-stranded DNA-binding protein to recognize a mismatch moiety (namely a single strand moiety) existing in the double-stranded nucleic acid and the phenomenon that the nucleic acid replication reaction is inhibited when the single-stranded DNA-binding protein is connected to the hybridization of a primer with the nucleic acid, that uses result of the nucleic acid amplification reaction (amplified amount) as the index.

The method of the invention includes:
(a) a step for obtaining an amplification reaction solution by allowing optional nucleic acids, primers and reagents capable of amplifying said target nucleic acid and a single-stranded DNA-binding protein to contact with one another;
(b) a step for amplifying the nucleic acid by elongating said primers; and
(c) a step for judging the presence or absence of a mismatch between the control nucleic acid and target nucleic acid by detecting whether or not the nucleic acids were amplified.

Timing of the contact of the single-stranded DNA-binding protein with DNA is not particularly limited, but it is desirable to carry it out after formation of a double-stranded nucleic acid which is described later.

The principle of the method of the invention is described in the following.

Firstly, a nucleic acid as the object of judging whether or not a mismatch is present is prepared. The nucleic acid may be an artificially synthesized sequence as a primer or the like or a sequence derived from a natural resource.

For example, genome and the like extracted from human blood, body fluids, tissues or cells, bacteria and viruses can be suitably used.

Next, primers and reagents capable of amplifying said target nucleic acid and a single-stranded DNA-binding protein are allowed to contact with these nucleic acids. The primers are designed in advance such that they become complementary to respective alleles. When the target nucleic acid and primers are hybridized, the target nucleic acid and primers form a complete double strand only when their genetic type coincided with one another. When did not coincide, the target nucleic acid and primers become partially single-stranded states. The single-stranded DNA-binding protein recognizes these single-stranded states and specifically binds to the sites.

Next, amplification of the double-stranded nucleic acid is carried out. When the nucleic acid amplification method is employed under the single-stranded DNA-binding protein-bonded state, the amplification reaction is suppressed. On the other hand, the amplification is generated when the single-stranded DNA-binding protein is not bonded.

Alternatively, as shown below, a target nucleic acid having a possibility of having a mutation and a control nucleic acid (nucleic acid having no mutation) may be prepared in advance and use them by hybridizing with each other. When the target nucleic acid has a mutation as a result of the hybridization, a hetero double-stranded nucleic acid (double-stranded nucleic acid having a mismatch) is formed by its hybridization with the control nucleic acid. On the other hand, when there is no mutation in the target nucleic acid, only a homo double-stranded nucleic acid (double stranded nucleic acid having no mismatch) is formed and the hetero double-stranded nucleic acid is not formed. When the single-stranded DNA-binding protein is allowed to contact with the double-stranded nucleic acid, the single-stranded DNA-binding protein binds to the hetero double-stranded nucleic acid having a mismatch but does not bind to the homo double-stranded nucleic acid.

Next, amplification of the double-stranded nucleic acid is carried out. When the nucleic acid amplification method is applied under a state in which the single-stranded DNA-binding protein is bonded to the hetero double-stranded nucleic acid, the amplification reaction is suppressed. On the other hand, amplification occurs in the homo double-stranded nucleic acid to which the single-stranded DNA-binding protein is not bonded.

Accordingly, whether or not the double-stranded nucleic acid in a sample has a mismatch can be judged in both cases by examining the presence or absence of the amplification of the double-stranded nucleic acid. In the detection of SNPs, whether or not the target nucleic acid has a mismatch. More illustratively, the presence or absence of a mismatch can be judged with more higher accuracy by examining amplified amounts of the double-stranded nucleic acid in the absence and presence of the single-stranded DNA-binding protein.

According to the invention, the "mismatch" means that a set of base pair selected from adenine (A), guanine (G), cytosine (C) and thymine (T) (uracil (U) in the case of RNA) is not a normal base pair (A/T or G/C). According to the invention, not only one mismatch but also continued two or more mismatches, a mismatch generated by the insertion and/or deletion of one or two or more bases and a combination thereof are included in the "mismatch".

According to the invention, the "mutation" indicates a base in the target double-stranded nucleic acid, which is different when compared with the case of the control double-stranded nucleic acid (a base pair in the case of the double-stranded nucleic acid, however, a case in which one of them is absent is also included), and means that it includes substitution of a base, deletion of a base and insertion of a base.

According to the invention, the "nucleic acid" is not particularly limited, and any one of the DNA and RNA or other artificial nucleic acids (so-called PNA and the like) can be used, for example, it may be a cDNA, genomic DNA, mRNA or the like natural sample or a synthetic polynucleotide. Also, it includes a double-stranded nucleic acid, a straight chain nucleic acid and a cyclic nucleic acid. Regarding the SNPs of a single-stranded nucleic acid, it can be inspected by hybridizing with a control nucleic acid.

According to the invention, the "control nucleic acid" means a nucleic acid which does not have a mutation. In addition, the "target nucleic acid" means a nucleic acid having a possibility of possessing a base different from the control nucleic acid (mutation). The target nucleic acid is a nucleic acid identical to the control nucleic acid when it does not have a mutation and is a nucleic acid wherein only said mutation site is different from the control nucleic acid when it has a mutation. For example, when a mutation in a gene of a patient having a possibility of suffering from a hereditary disease is detected, a gene of the patient having a possibility off possessing a mutation is the target nucleic acid and a healthy person's gene which corresponds to this gene is the control nucleic acid.

The target nucleic acid to be used in the method of the invention is not particularly limited, and any nucleic acid desired to be detected on whether not it has a mutation can be used. In addition, when the control nucleic acid is a nucleic acid which corresponds to a target nucleic acid and when the target nucleic acid does not have a mutation, a nucleic acid identical to the target nucleic acid can be used. This identical means that both of them are identical regarding the regions to be hybridized, and their lengths may be different but it is desirable to make the length uniform when possible. Each of the target nucleic acid and control nucleic acid may be a single chain or double chain.

The method of the invention can be suitably applied to the detection of a mutation in nucleic acid sequences such as a single mismatch base pair, two or more continued mismatches, a mismatch of one base pair two or more bases and a mismatch generated by the deletion ad/or insertion of one or two or more bases in at least one side chain of a double-stranded nucleic acid.

The single-stranded DNA-binding protein to be used in the method of the invention is a protein which acts in the replication process of DNA.

In its replication, the double helix of DNA is temporarily loosened from the replication origin, and new polynucleotide chains are synthesized using the thus exposed single strands as the respective templates. A substance which binds to the single strand moiety at this time to prevent returning of the single-stranded DNA to the double strand is the single-stranded DNA-binding protein.

Such a single-stranded DNA-binding protein (SSB) is an optional SSB conventionally known in this field. Preferably, it is a single-stranded DNA-binding protein (derived from *Escherichia coli, Drosophila* or *Xenopus*), a T4 bacteriophage-derived 32, 41, 44, 45 or 61 gene protein or an RPA protein in a eukaryote. In addition, the corresponding substances derived from other species can also be used preferably.

In addition, the single-stranded DNA-binding protein may be a protein (a mutant) including an amino acid sequence in which one or two or more amino acids in a natural type protein amino acid sequence is substituted, deleted and/or inserted, so far as it has the aforementioned function. Such a mutant may occur in the natural world, but it is possible to artificially prepare by optionally employing a conventionally known method.

It is possible to prepare the single-stranded DNA-binding protein as a natural protein or as a recombinant protein, by optionally combining an anion exchange column, a cation exchange column or a gel filtration column chromatography, an ammonium sulfate fractionation and the like conventionally known method. In addition, when it is a recombinant protein having high expression, it can also be possible to easily prepare only by a chromatography using a cation exchange column and a gel filtration column. A commercially available article can also be used as the single-stranded DNA-binding protein. Amount of the single-stranded DNA-binding protein to be used is generally from 1 to 50 µg, preferably from 3 to 20 µg, based on 1 µg of nucleic acid in a sample.

As the primers and reagents capable of amplifying a double-stranded nucleic acid, those which are used in the conventionally known nucleic acid amplification methods, such as the LAPM amplification method (Loop-Mediated Isothermal Amplification of DNA; Bio Industry, vol. 2, no. 2 (2001)), the RCA method (Rolling Circle Amplification; Proc. Natl. Acad. Sci., vol. 92, 4641 - 4645 (1995)), the ICAN method (Isothermal and Chimeric primer-initiated Amplification of Nucleic Acids), the SDA method (Strand Displacement Amplification (JP-A-5-130870), the NASBA method (Nucleic acid Sequence-based Amplification method; Nature, 350, 92 - (1991))-, TMA (Transcription mediated amplification method; J. Clin. Microbiol., vol. 31, 3270 - (1993)) and the like isothermal amplification reactions, general PCR amplification reaction and the like polymerase amplification reactions, LCR and the like (JP-A-5-2934) ligase amplification reactions and the like, can be used.

Illustratively, these are nucleic acids of primers designed such that they fitted to respective methods, a polymerase and/or a ligase (preferably Taq polymerase and the like thermostable enzymes), nucleic acid substrates (dNTP of dATP/dTTP(dUTP)/dCTP/dGTP), a buffer (e.g., Tris-SO₄ or the like) and a stabilizer (e.g., MgCl₂), a side reaction inhibitor (e.g., RNase) and the like, and can be used by the same kinds, amounts and methods of the conventional nucleic acid amplification. A commercially available nucleic acid amplification kit and the like can also be used.

The polymerase to be used in the nucleic acid amplification reaction may be any substance which has a strand displacement activity (strand-displacing ability), and any one of psychrophilic, mesophilic and thermophilic counterparts can be suitably used. Also, this polymerase may be anyone of the natural substances or artificially mutated mutants. As such a polymerase, a DNA polymerase can be exemplified. In addition, it is desirable that this DNA polymerase does not substantially have 5'→3' exonuclease activity. As such a DNA polymerase, 5'→3' exonuclease activity-deleted mutants of the DNA polymerase derived from *Bacillus stearothermophilus* (to be referred to as "B. st" hereinafter), *Bacillus* caldotenax (to be referred to as "B. ca" hereinafter) and the like thermophilic bacteria belonging to the genus *Bacillus,* Klenow fragment of *Escherichia coli* (*E. coli*)-derived DNA polymerase I and the like can be exemplified. As the DNA polymerase to be used in the nucleic acid amplification reaction, Vent DNA polymerase, Vent (Exo-) DNA polymerase, Deep Vent DNA polymerase, Deep Vent (Exo-) DNA polymerase, Φ 29 phage DNA polymerase, MS-2 phage DNA polymerase, Z-Taq DNA polymerase, Pfu DNA polymerase, KOD DNA polymerase, 9° Nm DNA polymerase, Therminater DNA polymerase and the like can be further exemplified.

It is desirable that the primers are designed in such a manner that they contain the periphery of a mismatch of object to be detected or the mismatch, and two or more of them can be used in response to the amplification method to be used. In addition, it is possible to design in such a manner that an optional primer of the primers to be used contains the periphery of a mismatch of object to be detected or the mismatch.

Regarding the primer designed in such a manner that it contains the periphery of a mismatch of object to be detected or the mismatch, a primer is designed such that a mismatch is contained preferably in the primer, or the mismatch is contained more preferably in the periphery of the polymerase reaction initiation terminal (within 1 to 20 bases from the 3'-end), further preferably within 1 to 10 bases from the 3'-end, most preferably within 1 to 5 bases from the 3'-end.

The contact of a double-stranded nucleic acid with the single-stranded DNA-binding protein in the method of the invention is carried out under such conditions that said protein can bind to a mismatch region in said double-stranded nucleic acid (e.g., appropriate pH, solvent, ionic environment and temperature). Illustrative conditions of the reaction temperature, salt concentration, kinds of ions, pH of a buffer and the like can be optionally adjusted.

As the methods for detecting whether or not a nucleic acid was amplified, general methods which use detection of various staining with ethidium bromide, intercalator fluorescence dye and the like, UV absorption, radio isotope or pyrophosphate, detection by a prove and the like can be used. Judgment of the presence or absence by setting appropriate concentration, sensitivity and the like conditions is also suitable because of the convenience, and it is desirable to calculate the concentration in improving the accuracy.

In judging the presence or absence of a mismatch between nucleic acids, it is more desirable to judge it by comparing a system in which the presence of a mismatch is known (negative control) and a system in which the absence of a mismatch is known (positive control) with the system of nucleic acid in a sample.

According to the invention, when a target nucleic acid is hybridized with a control nucleic acid, the double-stranded nucleic acid becomes a mixture of a hetero double-stranded nucleic acid and a homo double-stranded nucleic acid in the case of the presence of a mutation in the target nucleic acid and becomes a homo double-stranded nucleic acid alone in the case of the absence of a mutation in the target nucleic acid.

As the denaturation method of a double-stranded nucleic acid, for example, a method in which pH of the solution is acidified or alkalified and a method in which temperature of the solution is increased can be cited. As the method for changing pH, a method for replacing by 0.1 M NaOH or 0.1 M HCl can for example be cited. Also, in the temperature rising method, it may be adjusted to a melting temperature (Tm) or more of the nucleic acid but about 95°C is generally used.

Hybridization of two single-stranded nucleic acids can be easily carried out by returning pH of the solution to neutral or gradually lowering the temperature to Tm or less. When it is considered that the single-stranded nucleic acids are remained during the process of forming the double-stranded nucleic acid by hybridization, it is desirable to remove the single-stranded nucleic acids for example by a column.

In order to examine whether or not a specific gene in a patient having a possibility of having a hereditary disease has a mutation, the method of the invention can be applied to the examination on whether or not the gene derived from the patient and the gene of a healthy person have the same nucleotide sequence. According to the method of the invention, it is possible to detect a mutation in wherever position of the target nucleic acid it is present, and the method is superior also from the viewpoint that information on the mutation site of the gene to be examined and kind of the mutation in advance is not required. In addition, by applying the method of the invention to a sequence artificially synthesized as a primer or probe, accuracy of the sequence can be further improved.

The kit of the invention for judging the presence or absence of a mutation in a nucleic acid sequence is not particularly limited with the proviso that the materials to be used in the aforementioned mutation detection method of the invention (buffer, target nucleic acid, primers, nucleic acid staining agent, single-stranded DNA-binding protein, water and the like) are contained in one or more containers by optionally selecting said materials.

### [Examples]

The following describes the invention in detail with reference to examples, but the invention is not limited thereto.

### <Example 1>

### Detection of one base mutation and effect of single-stranded DNA-binding protein

### (1) Preparation of nucleic acid sample liquid containing target nucleic acid fragment

A 100 ng portion of Human Genomic cDNA (mfd. by Clontech) was converted into single strand by heating it at 98°C for 3 min., and then amplification of the sequence in β-actin gene was carried out under the following conditions.

### <Primers>

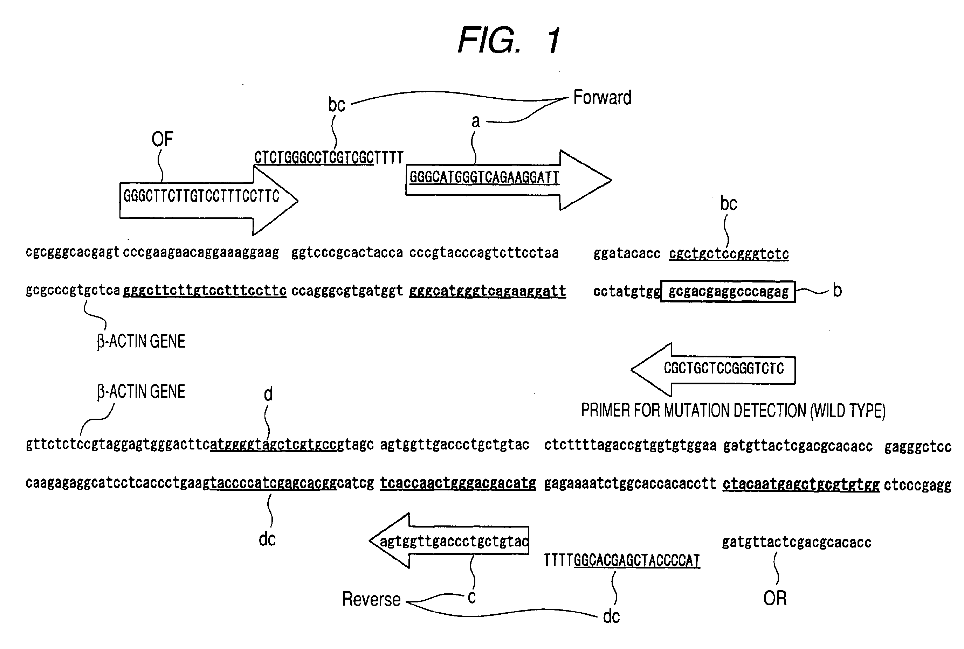

Primers were designed using the β-actin gene as the target. Physical relationship of respective primers is shown in Fig. 1. The forwardprimer (SEQ ID NO:1) (Forward) was designed such that the 3' -end region and 5' -end region shown by an arrow a complementary to the template is hybridized with the template region b existing in 10 bases downstream from the 3' -end base T on the elongation chain of the primer, and 4 bases of T were added between the linkage of the 5' -end of the aforementioned a and the 3'-end of a sequence identical to the region bc complementary to the region b. The reverse primer (SEQ ID NO:2) (Reverse) was designed such that the 3' -end region and 5' -end region shown by an arrow c complementary to the template is hybridized with the template region d existing in 6 bases downstream from the 3'-end base a on the elongation chain of the primer, and 4 bases of T were added between the linkage of the 5' -end of the aforementioned c and the 3' -end of a sequence identical to the region dc complementary to the region d.

Also, outer primers [OF (SEQ ID NO:3) and OR (SEQ ID NO:4)] were designed on the outside of the forward primer and reverse primer, respectively. In addition, in order to prepare a model system of one base mutation, a primer for mutation detection was newly prepared, and a primer having a sequence which matches with the template (SEQ ID NO:5, identical to the sequence bc) (wild type) and a primer having an artificial mutation by replacing its 3'-end C by T (SEQ ID NO:6) (mutation type) were prepared.

DNA sequences of respective primers are shown below.
Primer 1 (Forward)
5'-CTCTGGGCCTCGTCGCTTTTGGGCATGGGTCAGAAGGATT-3' (SEQ ID NO:1)
Primer 2 (Reverse) 5'-TACCCCATCGAGCACGGTTTTCATGTCGTCCCAGTTGGTGA-3' (SEQ ID NO:2)
Outer primer 3 (OF)
5'-GGGCTTCTTGTCCTTTCCTTC-3' (SEQ ID NO:3)
Outer primer 4 (OR)
5'-CCACACGCAGCTCATTGTAG-3' (SEQ ID NO:4)
Primer for mutation detection 5 (wild type)
5'-CTCTGGGCCTCGTCGC-3' (SEQ ID NO:5)
Primer for mutation detection 6 (mutation type)
5'-CTCTGGGCCTCGTCGT-3' (SEQ ID NO:6)

### (2) Nucleic acid amplification reaction

The amplification reaction was carried out by allowing a reaction liquid of the following composition to undergo the reaction at 60°C for 1 hour. The level 1 is a level in which the single-stranded DNA-bindingprotein was not added, and the levels 2 to 4 are levels in which the single-stranded DNA-binding protein was added by changing its concentration. Commercially available Bst. Polymerase (mfd. by NEB) was used as the synthase, and Single Stranded DNA Binding Protein (mfd. by Promega) as the single-stranded DNA-binding protein (SSB). SYBR Green I (mfd. by Takara Bio) was used in the nucleic acid staining.

**Table 1**

| <Composition of the reaction liquid> | | |
|---|---|---|
| | Level 1 | Levels 2 to 4 |
| 10 x Bst Buffer (DF) | 2.5 µl | the same as level 1 |
| 100 mM MgSO₄ | 1.5 µl | the same as level 1 |
| 10% (v/v) Tween 20 | 0.25 µl | the same as level 1 |
| 100% DMSO | 1.25 µl | the same as level 1 |
| 25 mM dNTP each | 1.4 µl | the same as level 1 |
| SYBR Green I (2000 times dilution) | | |
| | 0.5 µl | the same as level 1 |
| Primer 1 50 µM | 1.6 µl | the same as level 1 |
| Primer 2 50 µM | 1.6 µl | the same as level 1 |
| Primer 3 50 µM | 0.2 µl | the same as level 1 |
| Primer 4 50 µM | 0.2 µl | the same as level 1 |
| Primer 5 or 6 50 µM | 0.8 µl | the same as level 1 |
| Bst. Polymerase | 1.0 µl | the same as level 1 |
| Single-stranded DNA-binding protein | 0 µl | 1 µl |

| Nucleic acid fragment sample liquid | | |
|---|---|---|
| (100 ng) obtained (1) | 1.0 µl | the same as level 1 |
| Purified water | 11.2 µl | 10.2 µl |
| Total | 25.0 µl | 25.0 µl |

Added amounts of the single-stranded DNA-binding protein are as follows.
Level 1: 0
Level 2: 0.18 µg
Level 3: 0.54 µg
Level 4: 1.08 µg

### (3) Detection of amplification product

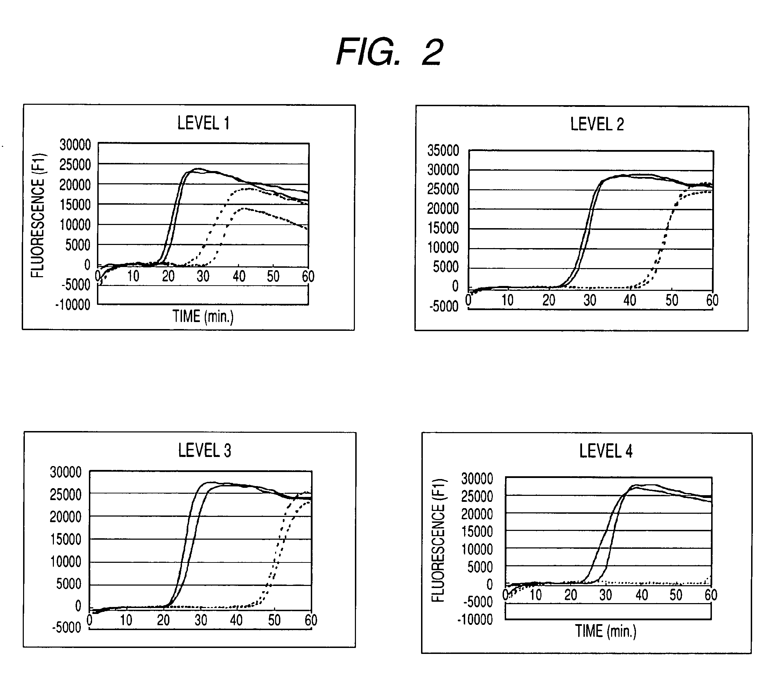

Fluorescence detection of the amplification reaction in the aforementioned (3) was carried out using a real time fluorescence detector (Mx3000p, mfd. by Stratagene). The results are shown in Fig. 2. The thick line is a case of using the wild type primer, and the dotted line a case of using the mutation type primer.

It can be seen that the amplification was started from the wild primer at about 20 minutes at all of the levels 1 to 4. In addition, regarding the levels 2 to 4 in the case of the addition of the single-stranded DNA-binding protein (SSB), it can be seen that the amplification from the mutant primer delayed in response to the added amounts of the single-stranded DNA-binding protein. Particularly regarding the level 4, it did not occur even after 60 minutes. It can be understood that nonspecific amplification is suppressed by the single-stranded DNA-binding protein. In this connection, period of times when the fluorescence quantity reached 250 in the aforementioned graph were calculated using the analyzing software of Mx3000p. The results are shown in the following table. The unit is minute and an average of n = 2.

**Table 2**

| | Added amount of SSB | Wild type | Mutation type |
|---|---|---|---|
| Level 1 | 0 µg | 17.0 | 28.5 |
| Level 2 | 0.18 µg | 22.5 | 41.2 |
| Level 3 | 0.54 µg | 20.7 | 43.3 |
| Level 4 | 1.08 µg | 24.1 | 57.7 |

It can certainly be seen from the above table that the amplification of the mutation type is suppressed in response to the added amount of SSB.

### <Example 2>

### Detection of SNPs (Arg16Gly) in human ADRB 2 gene

### (1) Preparation of nucleic acid sample liquid containing target nucleic acid fragment

Blood samples were collected in advance from agreed two healthy persons, and genomes were extracted using QuickGene DNA whole blood kit (mfd. by FUJIFILM Corporation). As a result of the analysis of these sequences, it was revealed that the healthy person A is a wild type homo and the healthy person B is a mutation type homo. A 100 ng portion of each of these genomes of two persons was converted into single strand by heating it at 98°C for 3 min., and then judgment of SNPs in the ADRB 2 gene was carried out under the following conditions.

### <Primers>

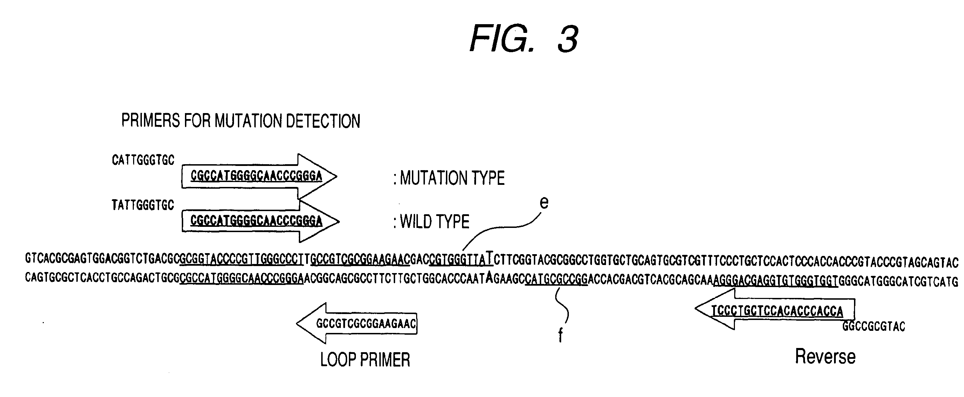

Primers were designed in such a manner that the LAMP method can be carried out using the ADRB 2 gene as the target. By the same standpoint of Fig. 1, each of the forward prime and reverse primer includes a 5'-end region designed in such a manner that the 3'-end region and 5'-end region complementary to the template are hybridized with a region on the elongation chain of the primer. Physical relationship of respective primers is shown in Fig. 3. In this connection, the sequence outside the arrow-shaped frame of the primer for mutation detection (wild type) is the same as the sequence e, and in the primer for mutation detection (mutation type), 5'-end T of the wild type is replaced by C. In this connection, the sequence outside the arrow-shaped frame of the reverse primer is the same as the sequence f.

DNA sequences of respective primers are shown below.
Primer for mutation detection 7 (wild type)
5'-TATTGGGTGCCGCCATGGGGCAACCCGGGA-3' (SEQ ID NO:7)
Primer for mutation detection 8 (mutation type)
5'-CATTGGGTGCCGCCATGGGGCAACCCGGGA-3' (SEQ ID NO:8)
Primer 9 (Reverse)
5'-CATGCGCCGGACCACCCACACCTCGTCCCT-3' (SEQ ID NO:9)
Loop Primer 10
5'-CAAGAAGGCGCTGCCG-3' (SEQ ID NO:10)

### (2) Nucleic acid amplification reaction

The amplification reaction of the genomes of two persons, sample A and sample B, was carried out by allowing a reaction liquid of the following composition to undergo the reaction at 60°C for 1 hour. The level is 2 levels for each sample, and one is a level which contains Single Stranded DNA Binding Protein (mfd. by Promega, 0.18 µg) and the other is a level which does not contain the same.

**Table 3**

| <Composition of the reaction liquid> | | |
|---|---|---|
| | Level 1 | Level 2 |
| 10 x Bst Buffer (DF) | 2.5 µl | the same as level 1 |
| 100 mM MgSO₄ | 1.5 µl | the same as level 1 |
| 10% (v/v) Tween 20 | 0.25 µl | the same as level 1 |
| 100% DMSO | 1.25 µl | the same as level 1 |
| 25 mM dNTP each | 1.4 µl | the same as level 1 |
| SYBR Green I (2000 times dilution) | | |
| | 0.5 µl | the same as level 1 |
| Primer 7 or 8 50 µM | 1.6 µl | the same as level 1 |
| Primer 9 50 µM | 1.6 µl | the same as level 1 |
| Primer 10 50 µM | 0.8 µl | the same as level 1 |
| Bst. Polymerase | 1.0 µl | the same as level 1 |
| Single-stranded DNA-binding protein | 1 µl | 0 µl |

| Nucleic acid fragment sample liquid | | |
|---|---|---|
| (100 ng) obtained (1) | 1.0 µl | the same as level 1 |
| Purified water | 10.6 µl | 11.6 µl |
| Total | 25.0 µl | 25.0 µl |

### (3) Judgment of genetic polymorphism

Fluorescence detection of the amplification reaction in the aforementioned (2) was carried out using a real time fluorescence detector (Mx3000p, mfd. by Stratagene). By carrying out the amplification using the wild type (wild) and mutation type (mutant) primers of each level, the genetic polymorphism was judged based on the presence or absence of their amplification.

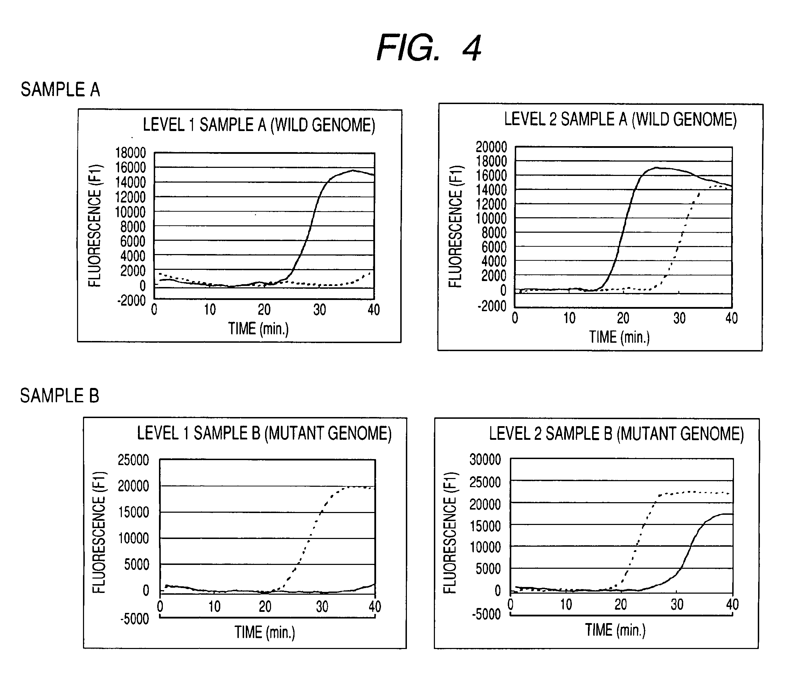

The results are shown in Fig. 4. The thick line is a case of using the wild type primer, and the dotted line a case of using the mutation type primer.

Both of the sample A and sample B coincided with known types (sample A: wild type, sample B: mutation type). In both of the samples, nonspecific amplification was suppressed by the level 1 in comparison with the level 2 (a level of not adding SSB), so that the genetic polymorphism can be distinctively typed.

### <Example 3>

### Comparison with a mutation recognizing protein (MutS)

### (1) Preparation of nucleic acid sample liquid containing target nucleic acid fragment

Using 100 ng of the genome of sample A used in Example 2 (SNPs (Arg16Gly) of ADRB 2 is wild type), it was converted into single strand by heating it at 98°C for 3 min., and then judgment of SNPs in the ADRB 2 gene was carried out under the following conditions using SSB and MutS as proteins which suppress nonspecific amplification.

### <Primers>

The same primers of Example 2 were used as the primers. That is, they are as follows.
Primer for mutation detection 7 (wild type)
5'-TATTGGGTGCCGCCATGGGGCAACCCGGGA-3' (SEQ ID NO:7)
Primer for mutation detection 8 (mutation type)
5'-CATTGGGTGCCGCCATGGGGCAACCCGGGA-3' (SEQ ID NO:8)
Primer 9 (Reverse)
5'-CATGCGCCGGACCACCCACACCTCGTCCCT-3' (SEQ ID NO:9)
Loop Primer 10
5'-CAAGAAGGCGCTGCCG-3' (SEQ ID NO:10)

### (2) Nucleic acid amplification reaction

The amplification reaction was carried out by allowing a reaction liquid of the following composition to undergo the reaction at 60°C for 1 hour. The level 1 is a level in which a single-stranded DNA-binding protein was not added, while MutS protein (mfd. by Nippon Gene) was added in the level 2, and Single Stranded DNA Binding Protein (mfd. by Promega) in the level 3. Commercially available Bst. Polymerase (mfd. by NEB) was used as the synthase.

**Table 4**

| <Composition of the reaction liquid> | | |
|---|---|---|
| | Level 1 | Levels 2 and 3 |
| 10 x Bst Buffer (DF) | 2.5 µl | the same as level 1 |
| 100 mM MgSO₄ | 1.5 µl | the same as level 1 |
| 10% (v/v) Tween 20 | 0.25 µl | the same as level 1 |
| 100% DMSO | 1.25 µl | the same as level 1 |
| 25 mM dNTP each | 1.4 µl | the same as level 1 |
| SYBR Green I (2000 times dilution) | | |
| | 0.5 µl | the same as level 1 |
| Primer 1 50 µM | 1.6 µl | the same as level 1 |
| Primer 2 50 µM | 1.6 µl | the same as level 1 |
| Primer 3 50 µM | 0.2 µl | the same as level 1 |
| Primer 4 50 µM | 0.2 µl | the same as level 1 |
| Primer 5 or 6 50 µM | 0.8 µl | the same as level 1 |
| Bst. Polymerase | 1.0 µl | the same as level 1 |

| MutS (level 2) or SSB (level 3) | | |
|---|---|---|
| | 0 µl | 1 µl |

| Nucleic acid fragment sample liquid | | |
|---|---|---|
| (100 ng) obtained (1) | 1.0 µl | the same as level 1 |
| Purified water | 10.2 µl | 11.2 µl |
| Total | 25.0 µl | 25.0 µl |

Added amount of the single-stranded DNA-binding protein was 0.18 µg and added amount of MutS was 1.0 µg.

### (3) Judgment of genetic polymorphism

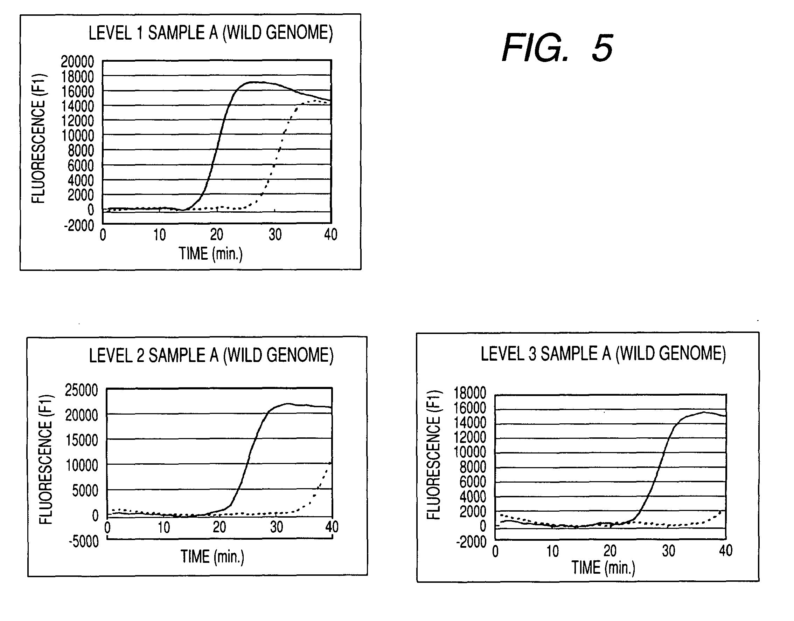

Fluorescence detection of the amplification reaction in the aforementioned (2) was carried out using a real time fluorescence detector (Mx3000p, mfd. by Stratagene). By carrying out the amplification using the wild type (wild) and mutation type (mutant) primers of each level, the genetic polymorphism was judged based on the presence or absence of their amplification. The results are shown in Fig. 5. The thick line is a case of using the wild type primer, and the dotted line a case of using the mutation type primer.

It can be seen that nonspecific amplification (amplification of the mutant side) was suppressed by both of MutS (level 2) and SSB (level 3) in comparison with the level 1. However, the added amount of MutS was 1.0 µg, while the added amount of SSB was 0.18 µg. The SSB shows similar or superior effect to suppress nonspecific amplification by 1/5 or less of the added amount of MutS which is a mutation recognizing protein. In this connection, period of times when the fluorescence quantity reached 250 in the aforementioned graph were calculated using the analyzing software of Mx3000p. The results are shown in the following table. The unit is minute.

**Table 5**

| | Added substance | Wild type | Mutation type |
|---|---|---|---|
| Level 1 | nothing | 15.9 | 24.7 |
| Level 2 | MutS | 18.3 | 32.1 |
| Level 3 | SSB | 22.5 | 36.4 |

It can be seen that SSB certainly has the suppressing ability of similar to or larger than that of MutS.

According to the invention, the presence or absence of a mismatch possessed by SNPs and the like double-stranded nucleic acid can be detected more efficiently and more accurately. The method of the invention can be applied to genetic diagnosis, infection diagnosis, genomic drug creation and the like uses.

The entire disclosure of each and every foreign patent application from which the benefit of foreign priority has been claimed in the present application is incorporated herein by reference, as if fully set forth.
Fig. 1
   #1: β-Actin gene
   #2: Primer for mutation detection (wild type)
Fig. 2
   #3: Level 1
   #4: Level 2
   #5: Level 3
   #6: Level 4
Fig. 3
   #7: Primers for mutation detection
   #8: Mutation type
   #9: Wild type
   #10: Loop primer
Fig. 4
   #11: Sample A
   #12: Level 1 Sample A (wild genome)
   #13: Level 2 Sample A (wild genome)
   #14: Sample B
   #15: Level 1 Sample B (mutant genome)
   #16: Level 2 Sample B (mutant genome)
Fig. 5
   #17: Level 1 Sample A (wild genome)
   #18: Level 2 Sample A (wild genome)
   #19: Level 3 Sample A (wild genome)

## Claims

1. A method for judging presence or absence of a mutation in a nucleic acid sequence, the method comprising:
utilizing a single-stranded DNA-binding protein.

2. The method according to claim 1, comprising:
(a) obtaining an amplification reaction solution by allowing a target nucleic acid, a primer and a reagent capable of amplifying the target nucleic acid and the single-stranded DNA-binding protein to contact with one another;
(b) amplifying the target nucleic acid by elongating the primer; and
(c) judging presence or absence of a mismatch between a control nucleic acid and the target nucleic acid by detecting whether or not the target nucleic acid is amplified.

3. The method according to claim 1,
wherein the presence or absence of a mutation in a nucleic acid sequence is judged by a product formed by a nucleic acid amplification reaction utilizing the single-stranded DNA-binding protein.

4. The method according to claim 1,
wherein the presence or absence of a mutation in a nucleic acid sequence is judged by presence or absence of an amplification reaction based on an action of the single-stranded DNA-binding protein to suppress a non-specific reaction of the nucleic acid amplification reaction.

5. The method according to claim 2,
wherein the nucleic acid amplification reaction is isothermally carried out.

6. The method according to claim 2,
wherein the nucleic acid amplification reaction is carried out utilizing a strand displacement type polymerase.

7. The method according to claim 1,
wherein the single-stranded DNA-binding protein is an SSB derived from *Escherichia coli, Drosophila* or *Xenopus,* a T4 phage gene 32, 41, 44, 45 or 61 protein or a mixture of at least two species thereof.

8. The method according to claim 1,
wherein a series of actions of extracting, amplifying and detecting a nucleic acid from a substance to be tested is continuously carried out in a sealed space.

9. The method according to claim 8,
wherein the substance to be tested is selected from the group consisting of blood, body fluids, tissues, cells, bacteria and viruses.

10. A kit for judging presence or absence of a mutation in a nucleic acid sequence by the method according to claim 1.


SEQUENCE LISTING
<110> FUJIFILM Corporation
<120> Method for detection of nucleic acid mutation by using Single Stranded DNA
Binding Protein
<130> 206-05380
<160> 10
<210> 1
<211> 40
<212> DNA
<213> Artificial Sequence
<220>
<223> Description of Artificial Sequence: Synthetic DNA
<400> 1
ctctgggcct cgtcgctttt gggcatgggt cagaaggatt 40
<210> 2
<211> 41
<212> DNA
<213> Artificial Sequence
<220>
<223> Description of Artificial Sequence: Synthetic DNA
<400> 2
taccccatcg agcacggtttt tcatgtcgtc ccagttggtg a 41
<210> 3
<211> 21
<212> DNA
<213> Artificial Sequence
<220>
<223> Description of Artificial Sequence: Synthetic DNA
<400> 3
gggcttcttg tcctttcctt c 21
<210> 4
<211> 20
<212> DNA
<213> Artificial Sequence
<220>
<223> Description of Artificial Sequence: Synthetic DNA
<400> 4
ccacacgcag ctcattgtag 20
<210> 5
<211> 16
<212> DNA
<213> Artificial Sequence
<220>
<223> Description of Artificial Sequence: Synthetic DNA
<400> 5
ctctgggcct cgtcgc 16

<210> 6
<211> 16
<212> DNA
<213> Artificial Sequence
<220>
<223> Description of Artificial Sequence: Synthetic DNA
<400> 6
ctctgggcct cgtcgt                                                    16
<210> 7
<211> 30
<212> DNA
<213> Artificial Sequence
<220>
<223> Description of Artificial Sequence: Synthetic DNA
<400> 7
tattgggtgc cgccatgggg caacccggga                                     30
<210> 8
<211> 30
<212> DNA
<213> Artificial Sequence
<220>
<223> Description of Artificial Sequence: Synthetic DNA
<400> 8
cattgggtgc cgccatgggg caacccggga                                     30
<210> 9
<211> 30
<212> DNA
<213> Artificial Sequence
<220>
<223> Description of Artificial Sequence: Synthetic DNA
<400> 9
catgcgccgg accacccaca cctcgtccct                                     30
<210> 10
<211> 16
<212> DNA
<213> Artificial Sequence
<220>
<223> Description of Artificial Sequence: Synthetic DNA
<400> 10
caagaaggcg ctgccg                                                    16